# EUROPÄISCHE PATENTSCHRIFT

(11) **EP 0 327 092 B1**
(45) Veröffentlichungstag und Bekanntmachung des Hinweises auf die Patenterteilung: **31.01.1996**
(21) Anmeldenummer: 89101824.4
(22) Anmeldetag: 02.02.1989
(51) Int. Cl.: C07F 9/40, C08K 5/53, C07H 13/00

(54) **Phosphonate, Verfahren zu deren Herstellung und deren Verwendung als Flammschutzmittel in Kunststoffen**
Phosphonates, process for their preparation and their use as flame protectors in plastic materials
Phosphonates, procédé pour les préparer et leur utilisation comme protecteurs des flammes dans les matériaux plastiques

(30) Priorität: 02.02.1988 DE 3803030
(43) Veröffentlichungstag der Anmeldung: 09.08.1989
(73) Patentinhaber: DR. TH. BÖHME KG CHEM. FABRIK GMBH & CO., D-82538 Geretsried (DE)
(72) Erfinder: Klein, Reinhard Dr.Dipl.-Chem., D-8113 Kochel am See (DE); Balbach, Günter Dr.Dipl.-Chem., D-8190 Wolfratshausen (DE)
(74) Vertreter: Fehners, Klaus Friedrich, Dipl.-Ing., Dipl.-Wirtsch.-Ing.

(56) Entgegenhaltungen:
- EP-A- 0 012 351
- EP-A- 0 017 008
- EP-A- 0 029 172
- GB-A- 1 250 963
- CHEMICAL ABSTRACTS, Band 78, Nr. 7, 19. Februar 1973, Seite 506, Zusammenfassung Nr. 43615g, Columbus, Ohio, US; G. BORISOV et al.: "Preparation of bisphosphites and their addition to aldehydes and ketones", & IZV. OTD. KHIM. NAUKI, BULG. AKAD. NAUK. 1972, 5(2), 175-80

## Beschreibung

Die Erfindung betrifft neuartige Phosphonate sowie deren Herstellung und deren Verwendung als Flammschutzmittel in Kunststoffen.

Als Flammschutzmittel für Kunststoffe wird nachwievor im großen Umfange Antimontrioxid in Verbindung mit halogenhaltigen, insbesondere bromhaltigen Organica verwendet.

Die im Zusammenhang mit der Darstellung und Verarbeitung von Antimontrioxid bekannt gewordenen Gesundheitsrisiken haben eine weltweite Suche nach Ersatzstoffen eingeleitet. Obwohl Antimontrioxid, das in Staubform cancerogene Aktivität aufweist, durch Verwendung pastöser Formulierung für den Kunststoffhersteller problemlos zu handhaben ist, muß die Weiterverarbeitung von Kunststoffteilen wie Sägen, Bohren, Fräsen, weiterhin als problematisch erscheinen, da die Aufnahme staubförmiger Partikel bei solchen Arbeitsgängen nicht ausgeschlossen werden kann.

Die Suche nach Ersatzstoffen für Antimontrioxid wurde nicht zuletzt aufgrund der beschriebenen gesundheitlichen Probleme ab 1979 intensiviert, als Sb₂O₃ erstmals in die Liste der gesundheitsschädlichen Arbeitsstoffe (MAK-Liste) in die Kategorie III B eingestuft wurde. Es erden seither eine Reihe von Ersatzstoffen für die Kombination Antimontrioxid - halogenhaltige Organica angeboten, doch sind deren Wirkung bei mengengleicher Anwendung ungenügend, oder aber die Wirksubstanz ist unverhältnismäßig teuer in der Herstellung.

Im dem wirtschaftlich wichtigen Bereich der Elektro- und Elektronikindustrie erden Forderungen nach halogenfreien Flammschutzmitteln in zunehmendem Maße gestellt. Kunststoffe werden als ideale Nichtleiter in großem Stil als elektrisches Isoliermaterial verwendet. Der Forderung nach halogenfreien Flammschutzmitteln liegt die Gefahr zugrunde, daß Schäden in Elektronikbauteilen und deren Folgen, verursacht durch korrosive Brandgase, den eigentlichen Brandschaden bei weitem übersteigen können.

In der US-PS 37 89 091 und US-PS 38 49 368 werden cyclische Phosphonsäureester durch Umsetzung von Phosphonsäure-, Phosphorsäure-, oder Carbonsäureester mit einem bicyclischen Phosphit hergestellt.

In der Europäischen Patentanmeldung 0 237 135 ist ein Phosphonatkörper als Flammschutzmittel offenbart, der allerdings in Polymere wegen seines kristallinen Charakters nur mit Hilfe eines heißen Einwalzverfahrens eingearbeitet werden kann.

In der europäischen Patentanmeldung 0 017 008 sind oberflächenaktive Phosphonsäureester beschrieben, die als Emulgier- oder Dispersionsmittel oder als Netzmittel zur raschen Auflösung wasserlöslicher Polymerer in Wasser verwendet werden und sich in Polymer/Öl-Dispersionen und Wasser/Öl-Emulsionen von wasserlöslichen Polymerisaten als Flockungsmittel, z.B. bei der Wasseraufbereitung und als Retentionsmittel in der Papierindustrie eignen.

Aus der US-PS 23 52 549 ist eine Umesterung zwischen zwei unterschiedlichen Dialkylalkanphosphonaten bekannt.

Der Erfindung liegt die Aufgabe zugrunde, neue Verbindungen zu schaffen, die als Flammschutzmittel verwendbar sind sowie ein Verfahren zur Herstellung der Verbindungen.

Überraschenderweise haben sich die erfindungsgemäß hergestellten Verbindungen gemäß der nachstehend aufgezeigten Formeln trotz ihres relativ niedrigen Phosphorgehaltes als außerordentlich wirksam erwiesen, obwohl nach allgemeiner Auffassung ein guter Flammschutzeffekt bei phosphorhaltigen Verbindungen einem sehr hohen Phosphorgehalt zuzuschreiben ist.

Als flammhemmende Verbindungen sind solche der allgemeinen Formel: 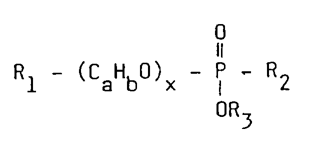 wobei: 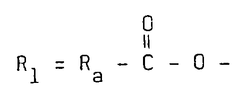 ein aromatischer Carboxylatrest, ein aliphatischer Carboxylatrest mit bis zu 7 Kohlenstoffatomen,
oder

R₁ = R_{b} - O

ein Phenolat, ein mit einem anderen Substituenten als Alkyl substituiertes Phenolat oder ein teilweise oder vollständig hydriertes Phenolat, ein Alkoholat eines gesättigten Alkoholes mit bis zu 7 Kohlenstoffatomen, ein Alkoholat eines ungesättigten Alkoholes mit bis zu 7 Kohlenstoffatomen, oder ein Monoalkoholat einer Di- oder Polyhydroxyverbindung bedeuten, wobei die Monoalkoholate von Ethylenglycol, Propylenglycol, Polyethylenglycol und Polypropylenglycol ausgenommen sind, soweit sie Reste eines Kohlenstoffkette mit mehr als 7 Kohlenstoffatomen enthalten, und R₂, R₃ einen Alkylrest (CₙH₂ₙ₊₁) mit n = 1, 2, 3, 4, repräsentieren und R₂ mit R₃ identisch sein kann;
wobei
- a =: 2 oder 3, b = 2a, x = ganze Zahl von 1-6, vorzugsweise 1 oder 2 im Falle von R_{a,}
- x =: 0 oder ganze Zahlen von 1-10 im Falle von R_{b} ist.

Bevorzugt sind dabei solche Phosphonate, bei denen:
der aromatische Carboxylatrest ein Benzoat-, Saliycylat- oder Kresotinatrest ist,
der aliphatische Carboxylatrest ein Acetat-, Propionat- oder Capronat-, rest ist,
das Alkoholat eines gesättigten Alkoholes der allgemeinen Formel CₙH₂ₙ₊₁ - O - mit n = 3-7, vorzugsweise n = 4-7 ist,
das Alkoholat eines ungesättigten Alkoholes der allgemeinen Formel CₙH₂ₙ₋₁ - O - mit n = 4-7 ist und bei denen R₁ auch ein Monoalkoholat von Di- oder Polyhydroxyverbindungen wie Sorbit, Bisphenol A, Zucker, Trimethylolpropan, Pentaerythrit, Polysaccharid oder Polyalkanolamin sein kann.

Ebenso wirksam haben sich Verbindungen der allgemeinen Formel: 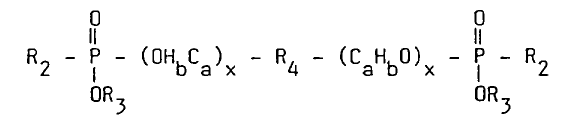 erwiesen, wobei:
R₄ ein Carboxylat einer Dicarbonsäure
oder
ein Dialkoholat einer Di- oder Polyhydroxyverbindung ist und
R₂, R₃ die gleiche Bedeutung wie in Anspruch 1 haben und
a = 2 oder 3, b = 2a
x = eine ganze Zahl von 1-6, vorzugsweise 1 oder 2 im Falle ist, daß R₄ ein Carboxylat einer Dicarbonsäure ist
x = 0 oder eine ganze Zahl von 1-10 ist, daß R₄ ein Dialkoholat einer Di- oder Polyhydroxy verbindung ist.

Bevorzugt sind hierfür solche Verbindungen bei denen R₄ ein Carboxylat einer Dicarbonsäure, wie Succinat, Oxalat, Maleinat, Fumarat, Adipinat, Phthalat, Terephthalat ist.

Ist R₄ ein Dialkoholat, so sind solche von Di- oder Polyhydroxyverbindungen wie beispielsweise Bisphenol A, Sorbit, Zucker, Polyalkylenglykole, Polysaccharide, Polyvinylalkohole, Polyalkanolamine, Hydrochinon, Resorcin, Trimethylolpropan oder Pentaerythrit bevorzugt.

Die vorstehend genannten Phosphonate sind aufgrund ihrer hohen thermischen Stabilität als Flammschutzmittel geeignet.

Gegenstand der Erfindung ist auch ein Verfahren zu Herstellung der Phosphonate. Hierbei wird ein vorgegebenes Dialkylalkanphosphonat mit hydroxylgruppenhaltigen Reaktionspartnern wie oxethylierten Carbonsäuren, mono- oder polyfunktionellen Alkoholen (oxalkyliert oder nicht oxalkyliert), Phenolen (oxalkyliert oder nicht oxalkyliert), Glykolen und Polyglykolen zur Reaktion gebracht, unter Bildung eines neues Phosphonates und Abspaltung des dem Ausgangsphosphonat zugrundeliegenden Alkoholes gemäß folgenden Reaktionsgleichungen 1 und 2:

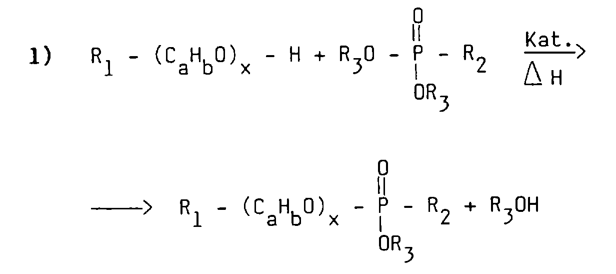

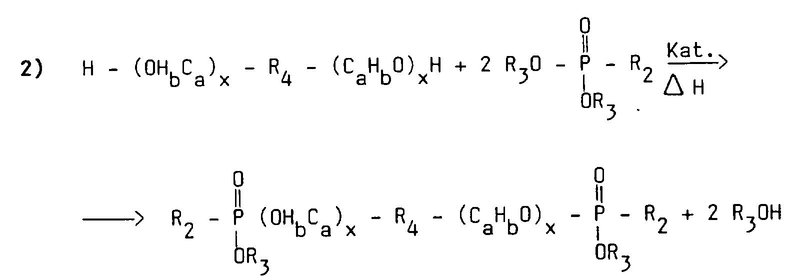

Die als Ausgangsverbindungen notwendigen Dialkylalkanphosphonate werden auf bekannte Weise durch Umlagerungsreaktionen nach Michaelis-Arbusov aus Trialkylphosphiten hergestellt (Lit. Houben-Weyl - Methoden der organischen Chemie, Band XII/1 S. 433 ff (1963)).

Die erfindungsgemäß eingesetzten Verbindungen werden durch Erhitzen von Esteroxalkylaten, Alkoholen und deren Oxalkylaten, Phenolen und deren Oxalkylaten, sowie Glykolen und Polyglykolen mit Phosphonsäureestern auf Rückflußtemperatur des Phosphonates hergestellt.

Höhere Reaktionstemperaturen, wie sie gegen Ende der Reaktion auftreten können, müssen vermieden werden, da dann eine Zersetzung der Ausgangsphosphonate nicht auszuschließen ist.

Als Katalysatoren haben sich Alkalien, wie Natrium- oder Kaliumhydroxid, Natrium- oder Kaliummethylat und Säuren, wie konzentrierte Schwefelsäure und p-Toluolsulfonsäure bewährt, bei Einsatzmengen von 0,05 - 1,0 Prozent, gemessen an der Gesamtmenge der Reaktionspartner.

Die nach dem vorstehenden Verfahren hergestellten neuen Phosphonate sind als Flammschutzmittel in Kunststoffen, vorzugsweise in Polyurethan, PVC, Polyester oder Epoxydharzen verwendbar.

Die erfindungsgemäß hergestellten neuen Phosphonate zeichnen sich durch überraschend hohe thermische Stabilität aus, was der beispielsweise in der US-PS 37 89 091 vertretenen Ansicht widerspricht, daß Phosphorverbindungen mit C-H-Bindungen an β-ständigen C-Atomen ab 200 °C zur Zersetzung unter Desalkylierung neigen. Die meisten der erfindungsgemäßen Verbindungen (Ausnahme sind Phosphonate aus Polyhydroxyverbindungen) sind bis etwa 250 °C thermisch stabil.

### Beispiel 1

In einen Dreihalskolben mit Rückflußkühler, KPG-Rührer und Stickstoffeinleitungsrohr werden 114,15 g (0,5 mol), Bisphenol A, 155,13 g (1,25 mol) Dimethylmethanphosphonat und 0,3 g Natriummethylat eingewogen. Der Rückflußkühler wird mit 70 °C warmem Wasser gespeist, auf seinem Kopf befindet sich eine mit Leitungswasser gekühlte Destillationsbrücke. Diese Apparatur ermöglicht das Abdestillieren von entstehendem Methanol und verhindert das Überdestillieren von Dimethylmethanphosphonat. Kurz nachdem unter einem Stickstoff-Polster die Siedetemperatur des DMMP erreicht ist (180 °C) setzt die Umesterungsreaktion ein, was an einer stetigen, gleichmäßigen Kondensation von Methanol in die Vorlage zu erkennen ist. Nach etwa 4 h ist die theoretische Menge von 40 ml Methanol (1 mol) überdestilliert. Unter Vakuum wird überschüssiges DMMP abgezogen.

Man erhält ein viskoses, schwach gelbes, klares Produkt.

### Beispiel 2

In der unter Beispiel 1 beschriebenen Apparatur werden 417,4 g Bisphenol A,2,4 EO (1,25 mol), 387,8 g DMMP (3,125 mol) und 0,8 g Natriummethylat auf 180 °C erhitzt. Nachdem 100,7 ml Methanol (2,5 mol) in die Vorlage überdestilliert sind, wird die Reaktion abgebrochen.

Man erhält ein schwach gelbes, klares, niedrigviskoses Produkt, von dem nach Bedarf überschüssiger Methanphosphonsäuredimethylester abdestilliert werden kann.

### Beispiel 3

In einen Dreihalskolben werden 97,4 g Maleinsäureoxethylat (2,2 EO) (0,5 mol), 155,1 g DMMP (1,25 mol) und 0,3 g Natriummethylat eingewogen. In der unter Beispiel 1 beschriebenen Weise wird die Reaktionsmischung 4 h unter Rückfluß gekocht, wobei die Reaktionstemperatur 190 °C nicht überschreitet. Dabei entstehen 40 ml Methanol (1,0 mol).

Das Reaktionsprodukt ist wasserklar, gelb und niedrigviskos. Überschüssiges DMMP kann nach Bedarf abdestilliert werden.

### Beispiel 4

In einen Dreihalskolben werden 150 g Polyethylenglykol 300 (0,5 mol) 155,1 g DMMP (1,25 mol) und 0,3 g Natriummethylat, wie in Beispiel 1 beschrieben, 3 h unter Rückfluß gehalten. Nachdem 40 ml Methanol in die Vorlage überdestilliert sind, wird abgekühlt.

Es entsteht ein niedrigviskoses, wasserklares, nahezu farbloses, klar wasserlösliches Produkt. Überschüssiges DMMP kann nach Bedarf abdestilliert werden.

### Beispiel 5

In einem Dreihalskolben werden 91,1 g Sorbit (0,5 mol), 155,1 g DMMP (1,25 mol) und 0,3 g Natriummethylat, wie in Beispiel 1 beschrieben, unter Rückfluß gehalten. Innerhalb von 3 h färbt sich die Reaktionsmischung hellbraun und die erwartete Menge von 40 ml Methanol (1 mol) ist in die Vorlage überdestilliert.

Man erhält ein hellbraunes, klares, wasserlösliches Produkt. Überschüssiges DMMP kann nach Bedarf abdestilliert werden.

### Beispiel 6

Flammschutzmittel gemäß Patentanspruch wurden in Polyurethan-Hartschäume folgender Rezeptur eingearbeitet:
- 33,95 %: polyfunktionelles Polyetherpolyol (OH-Zahl 395)
- 11,32 %: Monofluortrichlormethan
- 0,50 %: N-Ethylmorpholin
- 10,00 %: Umsetzungsprodukt aus Bisphenol A.2,4 EO und Dimethylmethanphosphonat aus Beispiel 2

Der homogenen Mischung dieser Komponenten werden
- 25,00 %: eines rohen, flüssigen 4,4-Diphenylmethandiisocyanates zugesetzt und kurz gerührt.

Der entstehende Schaum wird 14 Tage bei Normklima gelagert (20 °C, 65 % rel. Luftfeuchtigkeit - DIN 50 014) und nach DIN 4 102 Prüfverfahren B 2 geprüft. Sämtliche Proben bestehen des Test.

### Beispiel 7

Flammschutzmittel nach Beispiel 2 wird in folgende Rezeptur zur Herstellung eines Polyurethanweichschaumes eingearbeitet:
- 58,20 %: trifunktionelles Polyetherpolyol, MG 3500 (OH-Zahl 47,3)
- 0,24 %: Dibutylzinndilaurat
- 0,12 %: Diazabicyclooctan
- 0,44 %: Oxethyliertes Siliconöl (z.B. L 520 von Union Carbide)
- 2,10 %: Wasser
- 10,00 %: Flammschutzmittel nach Beispiel 2

Der Ansatz wird möglichst lange vermischt. Anschließend werden
- 28,90 %: Toluylendiisocyanat (technisches Isomerengemisch) zugesetzt und rasch verrührt.

Der entstehende Polyurethan-Weichschaum wird 14 Tage bei Normklima (DIN 50 014) gelagert. Sein Brandverhalten wird nach DIN 4 102 - Prüfverfahren B2 - getestet. Sämtliche Proben bestehen den Test.

### Beispiel 8

Flammschutzmittel nach Beispiel 2 wird in einen handelsüblichen Copolyester für die Verwendung als Schmelzkleber eingearbeitet.

Der Copolyester ist polykondensiert aus:
25 mol % Terephthalsäure
25 mol % Isophthalsäure
25 mol % Ethylenglykol
25 mol % Butandiol 1,4

100 Teile des Copolyesters in Pulverform werden mit 10 Teilen Flammschutzmittel nach Beispiel 2 gemischt, wobei zur Erzielung einer homogenen Mischung zunächst nur ca. 20 Teile Polyesterpulver zu 10 Teilen Flammschutzmittel gegeben werden und das restliche Polyesterpulver unter dauerndem Rühren nach und nach zugesetzt wird. Die erhaltene Masse wird in einer Metallform geschmolzen und durch Abkühlen zu einer Platte verarbeitet.

Eine Platte ohne und eine Platte mit Flammschutzmittel werden senkrecht aufgehängt und 15 sec. mit einem Bunsenbrenner kantenbeflammt.

Die Platte ohne Flammschutzmittel entzündet sich nach 5 sec. und brennt nach Entfernen des Bunsenbrenners weiter bis sie vollständig verbrannt ist, wobei brennendes Material abtropft. Die Platte mit eingearbeitetem Flammschutzmittel entzündet sich nicht. Während der Beflammung wird die Platte zwar weich und verformt sich, es fallen jedoch keine brennenden Tropfen herunter.

### Beispiel 9

Flammschutzmittel nach Beispiel 2 wird in eine PVC-Paste folgender Zusammensetzung eingearbeitet:

| | **Prüfling 1** | **2** | **3** |
|---|---|---|---|
| Emulsions PVC **VESTOLIT E 7012** | 100 | 100 | 100 |
| PVC-Weichmacher **VESTINOL N** | 60 | 50 | 40 |
| PVC-Weichmacher **SANTICIZER 160** | 20 | 10 | 5 |
| Stabilisator **NUOSTAB G 1014** | 2 | 2 | 2 |
| Flammschutzmittel nach Beispiel 2 | - | 20 | 25 |

Die PVC-Pasten werden mit einer Rakel (0,3 mm) auf Glasplatten aufgestrichen und bei 180 °C 3 min. lang geliert. Die entstandenen Folien werden zusammengerollt, senkrecht aufgehängt und 15 sec. mit dem Bunsenbrenner kantenbeflammt.

- **Prüfling 1**: entzündet sich rasch und brennt nach dem Entfernen des Bunsenbrenners unter Entwicklung schwarzen Rauches selbständig weiter.
- **Prüfling 2**: verlischt sofort nach dem Entfernen des Bunsenbrenners, wobei zunächst wenig weißer Rauch entsteht
- **Prüfling 3**: verlischt sofort nach dem Entfernen des Bunsenbrenners. Die Rauchentwicklung ist stärker als bei Prüfling 2.

### Beispiel 10

- 2,4: Gewichtsteile eines Bisphenol A-Epichlorhydrin-Harzes mit einem durchschnittlichen Molgewicht von 600 werden mit
- 0,3: Gewichtsteilen Flammschutzmittel nach Beispiel 2 verrührt. Zu dieser Mischung werden
- 0,6: Gewichtsteile eines Härters, bestehend aus 90 % Isophorondiamin und 10 % Benzylalkohol, gegeben und homogenisiert. Die erhaltene Masse wird mit Hilfe von Siliconkautschukformen zu 3 mm starken quadratischen Platten von 100 mm Kantenlänge gegossen. Nach einer Aushärtungszeit von 48 Stunden werden die Platten nach DIN 51 960 klimatisiert. Anschließend werden die Platten nach DIN 51 960 getestet.

| **PROBEN** | **1** | **2** | **3** |
|---|---|---|---|
| Flammschutzmittel nach | | | |
| Beispiel 2 in % | 0 | 10 | 15 |
| Längste Brennstrecke in mm | 35 (wird gelöscht, da nicht selbstverlöschend!) | 4 | 4 |
| Brandflecktiefe in mm | 3 | 1 | 0,5 |

Bei den Proben 2 und 3 erlischt die Flamme selbständig, sobald der Alkohol und der Zellstoff verbrannt sind.

## Patentansprüche

1. Verbindungen der allgemeinen Formel 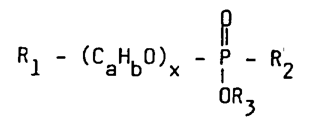 wobei 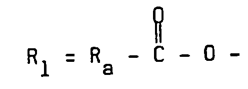 ein aromatischer Carboxylatrest; ein aliphatischer Carboxylatrest mit bis zu 7 Kohlenstoffatomen
oder
R₁ = R_{b} - 0 -
ein Phenolat, ein mit anderen Substituenten als Alkyl substituiertes Phenolat oder ein teilweise oder vollständig hydriertes Phenolat, ein Alkoholat eines gesättigten Alkoholes mit bis zu 7 Kohlenstoffatomen, ein Alkoholat eines ungesättigten Alkoholes mit bis zu 7 Kohlenstoffatomen oder ein Monoalkoholat einer Di- oder Polyhydroxyverbindung bedeuten, wobei die Monoalkoholate von Ethylenglycol, Propylenglycol, Polyethylenglycol und Polypropylenglycol ausgenommen sind, soweit sie Reste einer Kohlenstoffkette mit mehr als 7 Kohlenstoffatome enthalten,
und
R₂, R₃ einen Alkylrest (CnH₂ₙ₊₁) mit n = 1, 2, 3, 4 repräsentieren und R₂ mit R₃ identisch sein kann;
wobei
a = 2 oder 3, b = 2a, x = ganze Zahl von 1-6, vorzugsweise 1 oder 2 im Falle von Rₐ,
x = 0 oder ganze Zahlen von 1-10 im Falle von R_{b} ist.

2. Verbindungen nach Anspruch 1,
**dadurch gekennzeichnet**
daß der aromatische Carboxylatrest ein Benzoat, ein Salicylat oder ein Kresotinat ist.

3. Verbindungen nach Anspruch 1,
**dadurch gekennzeichnet**
daß der aliphatische Carboxylatrest ein Acetat, ein Propionat oder ein Capronat ist.

4. Verbindungen nach Anspruch 1,
**dadurch gekennzeichnet**
daß das Alkoholat eines gesättigten Alkoholes ein solches der allgemeinen Formel CₙH₂ₙ₊₁ - O - mit n = 3-7, vorzugsweise n = 4-7, ist.

5. Verbindungen nach Anspruch 1,
**dadurch gekennzeichnet**
daß das Alkoholat eines ungesättigten Alkoholes ein solches der allgemeinen Formel CₙH₂ₙ₋₁ - O - mit n = 4-7, ist.

6. Verbindungen nach Anspruch 1,
**dadurch gekennzeichnet**
daß das Monoalkoholat einer Di- oder Polyhydroxyverbindung sich von Sorbit, Bisphenol A, einem Zucker, Trimethylolpropan, Pentaerythrit, einem Polysaccharid oder einem Polyalkanolamin ableitet.

7. Verbindungen der allgemeinen Formel: 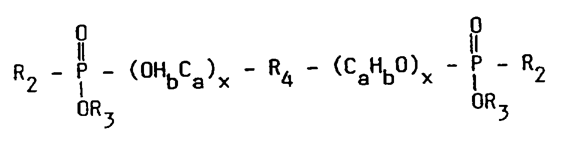 wobei
R₄
ein Carboxylat einer Dicarbonsäure
oder:
ein Dialkoholat einer Di- oder Polyhydroxyverbindung ist
und:
R₂, R₃ die gleiche Bedeutung wie in Anspruch 1 haben
und:
a = 2 oder 3, b = 2a
x = eine ganze Zahl von 1-6, vorzugsweise 1 oder 2 im Falle ist, daß R₄ ein Carboxylat einer Dicarbonsäure ist,
x = 0 oder eine ganze Zahl von 1-10 im Falle ist, daß R₄ ein Dialkoholat einer Di- oder Polyhydroxyverbindung ist.

8. Verbindungen nach Anspruch 7,
**dadurch gekennzeichnet**
daß das Carboxylat einer Dicarbonsäure ein Succinat, Oxalat, Maleinat, Fumarat, Adipinat, Phthalat, Terephthalat ist.

9. Verbindungen nach Anspruch 7,
**dadurch gekennzeichnet**
daß sich das Dialkoholat von einer Di- oder Polyhydroxyverbindung wie Bisphenol A, Sorbit, Zucker, Polyalkylenglykol, Polysaccharid, Polyvinylalkohol, Polyalkanolamin, Hydrochinon, Resorcin, Trimethylolpropan oder Pentaerythrit herleitet.

10. Verfahren zur Herstellung der Verbindungen nach Anspruch 1
**dadurch gekennzeichnet**,
daß Dialkylalkanphosphonate mit Hydroxylverbindungen nach folgenden Gleichungen umgeestert werden:

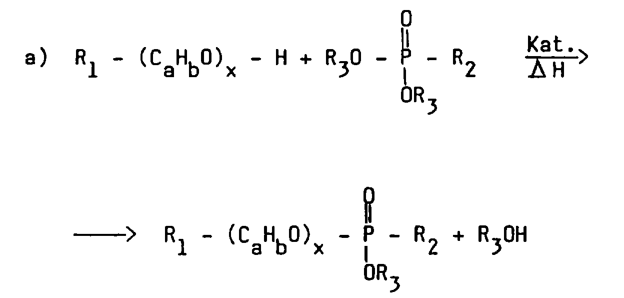

11. Verfahren zur Herstellung der Verbindungen nach Anspruch 7,
**dadurch gekennzeichnet**, daß Dialkylalkanphosphonate mit Hydroxylverbindungen nach folgenden Reaktionsgleichungen umgeestert werden :

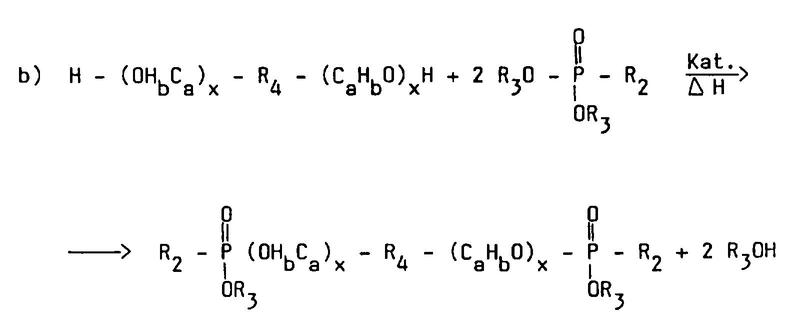

12. Verwendung der Phosphonate gemäß Anspruch 1-11 als Flammschutzmittel in Kunststoffen vorzugsweise in Polyurethan, PVC, Polyester und Epoxydharz.

## Claims

1. Compounds having the general formula 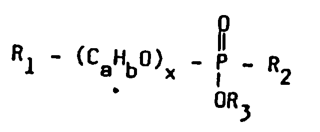 wherein 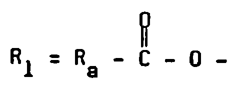
an aromatic carboxylate radical; an aliphatic carboxylate radical with up to 7 carbon atoms or
R₁ = R_{b}-0-
denote a phenolate, a phenolate substituted with substituents other than alkyl or a partly or completely hydrogenated phenolate, an alcoholate of a saturated alcohol with up to 7 carbon atoms, an alcoholate of an unsaturated alcohol with up to 7 carbon atoms or a monoalcoholate of a di- or polyhydroxy compound, the monoalcoholates of ethylene glycol, propylene glycol, polyethylene glycol and polypropylene glycol being excepted in so far as they contain radicals of a carbon chain with more than 7 carbon atoms, and
R₂, R₃ denote an alkyl radical (CₙH₂ₙ₊₁) with n = 1, 2, 3, 4 and R₂ can be identical with R₃ and
a = 2 or 3, b = 2a, x = an integer from 1 to 6, preferably 1 or 2 in the case of Rₐ, x = 0 or integers from 1 to 10 in the case of R_{b}.

2. Compounds according to claim 1, characterised in that the aromatic carboxylate radical is a benzoate, a salicylate or a cresotinate.

3. Compounds according to claim 1, characterised in that the aliphatic carboxylate radical is an acetate, a propionate or a capronate.

4. Compounds according to claim 1, characterised in that the alcoholate of a saturated alcohol has the general formula CₙH₂ₙ₊₁ -0- with n = 3-7, preferably n = 4-7.

5. Compounds according to claim 1, characterised in that the alcoholate of an unsaturated alcohol has the general formula CₙH₂ₙ₋₁ -0- with n = 4-7.

6. Compounds according to claim 1, characterised in that the monoalcoholate of a di- or polyhydroxy compound is derived from sorbitol, bisphenol A, a sugar, trimethylol propane, pentaerythritol, a polysaccharide or a polyalkanolamine.

7. Compounds having the general formula 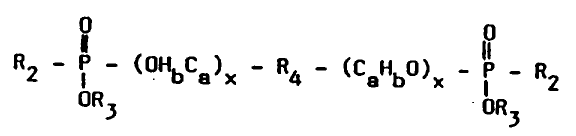 wherein
R₄ denotes a carboxylate of a dicarboxylic acid or
a dialcoholate of a di- or polyhydroxy compound and
R₂, R₃ have the same meaning as in Claim 1 and
a = 2 or 3, b = 2a
x = an integer from 1-6, preferably 1 or 2 in the case where R₄ is a carboxylate of a dicarboxylic acid and
x = 0 or an integer from 1 to 10 in the case where R₄ is a dialcoholate of a di- or polyhydroxy compound.

8. Compounds according to claim 7, characterised in that the carboxylate of a dicarboxylic acid is a succinate, oxalate, maleinate, fumarate, adipinate, phthalate or terephthalate.

9. Compounds according to claim 7, characterised in that the dialcoholate is derived from a di- or polyhydroxy compound such as bisphenol A, sorbitol, sugar, polyalkylene glycol, polysaccharide, polyvinyl alcohol, polyalkanolamine, hydroquinone, resorcinol, trimethylol propane or pentaerythritol.

10. A process for the preparation of the compounds according to claim 1, characterised in that dialkyl alkane phosphonates are transesterified with hydroxyl compounds in accordance with the following equations:

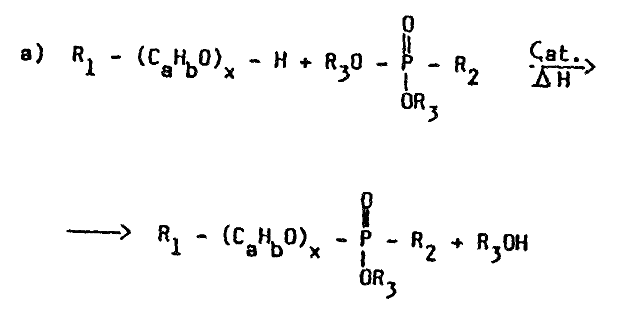

11. A process for the preparation of the compounds according to claim 7, characterised in that dialkyl alkane phosphonates are transesterified with hydroxyl compounds in accordance with the following reaction equations:

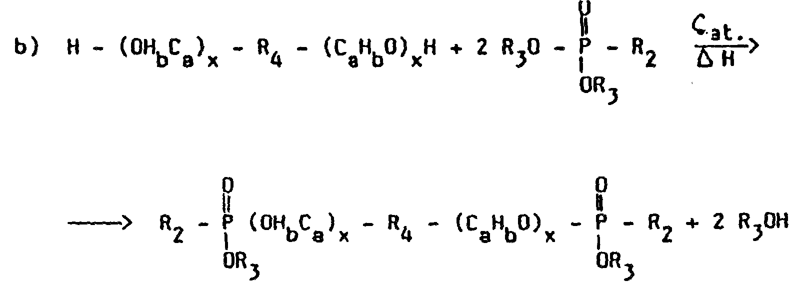

12. Use of the phosphonate according to claims 1 - 11 as flame retardants in plastics, preferably in polyurethane, PVC, polyester and epoxy resin.

## Revendications

1. Composés de formule générale 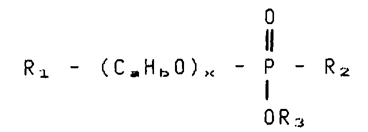 dans laquelle 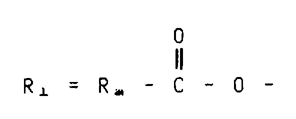 est un résidu carboxylat aromatique, un résidu carboxylate aliphatique comportant jusqu'à 7 atomes de carbone
ou
R₁ = R_{b} - 0 -
est un phénolate, un phénolate substitué avec d'autres substituants qu'un alkyle ou un phénolate partiellement ou complètement hydrogéné, un alcoolat d'un alcool saturé comportant jusqu'à 7 atomes de carbone, un alcoolat d'un alcool insaturé ayant jusqu'à 7 atomes de carbone ou un monoalcoolat d'un composé di- ou polyhydroxy, les monoalcoolat d'éthylène glycol, de propylène glycol, polyéthylène glycol et polypropylène glycol étant exclus, dans la mesure où ils contiennent des résidus d'une chaîne carbonée comportant plus de 7 atomes de carbone,
et
R₂, R₃ étant un résidu alkyle (CₙH₂ₙ₊₁) avec n = 1, 2, 3, 4 et R₂ pouvant être identique à R₃;
où en outre a = 2 ou 3, b = 2a, x est un nombre entier compris dans la plage allant de 1 à 6, de préférence 1 ou 2 dans le cas de Rₐ,
x = 0 ou des nombres entiers compris entre 1 à 10 dans le cas de R_{b}.

2. Composés selon la revendication 1, caractérisés en ce que le résidu carboxylate aromatique est un benzoate, un salicylate ou crésotinate.

3. Composés selon la revendication 1, caractérisés en ce que le résidu carboxylate aliphatique est un acétate, un propionate ou un capronate.

4. Composés selon la revendication 1, caractérisés en ce que l'alcoolat d'un alcool saturé est un alcoolat de formule générale CₙH₂ₙ₊₁ - 0 - avec n = 3 à 7, de préférence n = 4 à 7.

5. Composés selon la revendication 1, caractérisés en ce que l'alcoolat d'un alcool insaturé est un alcoolat de formule générale CₙH₂ₙ₋₁ - 0 - avec n = 4 à 7.

6. Composés selon la revendication 1, caractérisés en ce que le monoalcoolat d'un composé di- ou polyhydroxy est dérivé de la sorbite, du bisphénol A, d'un sucre, du triméthylolpropane, de la pentaérythrite, d'un polysaccharide ou d'un polyalkanolamine.

7. Composés de formule générale : 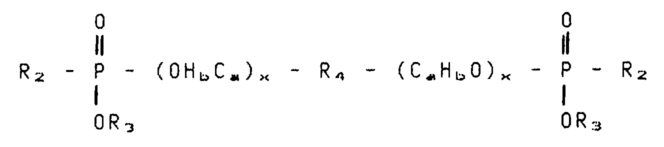 dans laquelle
R₄
est un carboxylate d'un acide dicarbonique
ou :
un dialcoolat d'un composé di- ou polyhydroxy
et :
R₂, R₃ ont la même signification que dans la revendication 1
et :
a = 2 ou 3, b = 2a,
x = un nombre entier compris entre 1 et 6, de préférence 1 ou 2 dans le cas où R₄ est un carboxylate d'un acide dicarbonique,
x = 0 ou un nombre entier compris entre 1 et 10 dans le cas où R₄ est un dialcoolat d'un composé di- ou polyhydroxy.

8. Composés selon la revendication 7, caractérisés en ce que le carboxylat d'un acide dicarbonique est un succinate, un oxalate, un maléinate, un fumarate, un adipinate, un phthalate, un térephthalate.

9. Composés selon la revendication 7, caractérisés en ce que le dialcoolat provient d'un composé di-ou polyhydroxy tel que le bisphénol A, la sorbite, le sucre, le polyalkylenglycol, le polysaccharide, le polyvinylalcool, le polyalkanolamine, hydrochinone, résorcine, triméthylolpropane ou pentaérythrite.

10. Procédé de préparation des composés selon la revendication 1, caractérisé en ce que les dialkylalcanophosphonate sont convertis avec des composés hydroxyles selon les formules ci-après :

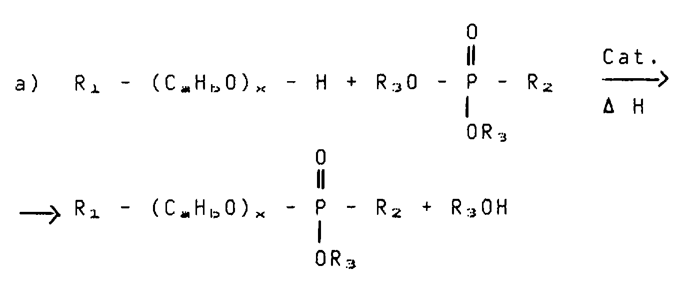

11. Procédé de préparation des composés selon la revendication 7, caractérisé en ce que les dialkylalcanophosphonate sont convertis avec des composés hydroxyles selon les formules de réaction ci-après

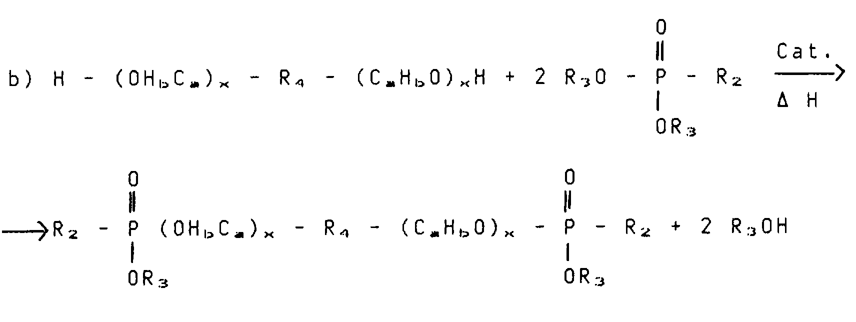

12. Utilisation des phosphonates selon les revendications 1 à 11 comme agents de protection contre la flamme dans des matières synthétiques, de préférence dans du polyuréthanne, du PVC, du polyester et de la résine époxyde.
